# EUROPÄISCHE PATENTSCHRIFT

(11) **EP 0 708 766 B1**
(45) Veröffentlichungstag und Bekanntmachung des Hinweises auf die Patenterteilung: **10.12.1997**
(21) Anmeldenummer: 94920981.1
(22) Anmeldetag: 09.07.1994
(51) Int. Cl.: C07D 311/16, C07D 311/18, A61K 31/37, C07D 493/04, C07D 405/06, C07D 215/22

(54) **VERWENDUNG VON CUMARINEN ALS PLA 2-INHIBITOREN, NEUE CUMARINE, VERFAHREN ZU IHRER HERSTELLUNG UND ARZNEIMITTEL**
USE OF COUMARINS AS PLA 2-INHIBITORS, NEW COUMARINS, PROCESS FOR PREPARING THE SAME AND MEDICAMENTS
UTILISATION DE COUMARINES COMME INHIBITEURS DE LA PLA 2, NOUVELLES COUMARINES,LEUR PROCEDE DE PREPARATION ET MEDICAMENTS

(30) Priorität: 13.07.1993 DE 4323409
(43) Veröffentlichungstag der Anmeldung: 01.05.1996
(73) Patentinhaber: BOEHRINGER MANNHEIM GMBH, 68298 Mannheim (DE)
(72) Erfinder: FRIEBE, Walter-Gunar, D-68165 Mannheim (DE); SCHAEFER, Wolfgang, D-68199 Mannheim (DE); SCHEUER, Werner, D-83646 Bad Tölz (DE); TIBES, Ulrich, D-60599 Frankfurt (DE)
(86) Internationale Anmeldenummer: EP9402251
(87) Internationale Veröffentlichungsnummer: WO9502588

(56) Entgegenhaltungen:
- EP-A- 0 164 040
- EP-A- 0 516 532
- EP-A- 0 614 896
- FR-A- 2 276 819
- FR-M- 2 035
- GB-A- 1 226 896
- CHEMICAL AND PHARMACEUTICAL BULLETIN., Bd.30, Nr.11, November 1982, TOKYO JP Seiten 4120 - 4125 AKIRA TAKADATE ET AL 'Synthesis and properties of 4-diazomethyl-7-methoxycoumarin as a new fluorescent reagent for alcohols and carboxylic acids'
- JOURNAL OF MEDICINAL CHEMISTRY, Bd.34, Nr.7, Juli 1991, WASHINGTON US Seiten 2268 - 2274 RICHARD J. HODGKISS ET AL 'Fluorescent markers for hypoxic cells'
- CHEMICAL ABSTRACTS, vol. 92, no. 5, 4. Februar 1980, Columbus, Ohio, US; abstract no. 41697a, JIRI KREJCOVES ET AL 'The preparation and characterization of some fluorescence labels derived from 7-substituted 2H-1-benzopyran-2-ones' Seite 761 ; & COLL. CZECH.CHEM. COMMUN., Bd.44, Nr.7, 1979 Seiten 2211 - 2220
- CHEMICAL ABSTRACTS, vol. 95, no. 17, 26. Oktober 1981, Columbus, Ohio, US; abstract no. 150350q, MANOHAR V. KULKARNI ET AL 'Studies on coumarins' Seite 639 ; & ARCH. PHARM., Bd.314, Nr.8, 1981, WEINHEIM Seiten 708 - 711
- CHEMICAL ABSTRACTS, vol. 98, no. 17, 25. April 1983, Columbus, Ohio, US; abstract no. 143233n, MANOHAR V. KULKARNI ET AL 'Studies on coumarins' Seite 561 ; & ARCH. PHARM., Bd.316, Nr.1, 1983, WEINHEIM Seiten 15 - 21
- CHEMICAL ABSTRACTS, vol. 100, no. 5, 30. Januar 1984, Columbus, Ohio, US; abstract no. 31674k, R. FARINNOTTI ET AL '4-Bromomethyl-6-7-dimethoxycoumarin as a fluorescent label for carboxylic acids in chromatographic detection' Seite 213 ; in der Anmeldung erwähnt & J. CHROMATOGR., Bd.269, Nr.2, 1983 Seiten 81 - 90
- CHEMICAL ABSTRACTS, vol. 103, no. 11, 16. September 1985, Columbus, Ohio, US; abstract no. 87858c, Seite 609 ; & JP,A,6 056 985 (SANKYO CO LTD) 2. April 1985
- CHEMICAL ABSTRACTS, vol. 103, no. 17, 28. Oktober 1985, Columbus, Ohio, US; abstract no. 141793t, SHRIKANT S. HANMANTGAD ET AL 'Synthesis and biological activity of 4-(sulfonamidomethyl)coumarins' Seite 706 ; & INDIAN J. CHEM.,SECT.B, Bd.24, Nr.4, 1985 Seiten 459 - 461
- CHEMICAL ABSTRACTS, vol. 104, no. 19, 12. Mai 1986, Columbus, Ohio, US; abstract no. 168321v, SHRIKANT S. HANMANTGAD ET AL 'Synthesis and some biomimetic 4-substituted coumarins' Seite 658 ; & REV. ROUM. CHIM., Bd.30, Nr.8, 1985 Seiten 735 - 741

## Beschreibung

Gegenstand der vorliegenden Erfindung ist die Verwendung von Cumarinen als PLA₂-Inhibitoren, neue Cumarinderivate, Verfahren zu deren Herstellung und Arzneimittel, die diese Verbindungen enthalten.

Die Erfindung betrifft Cumarinderivate der allgemeinen Formel I 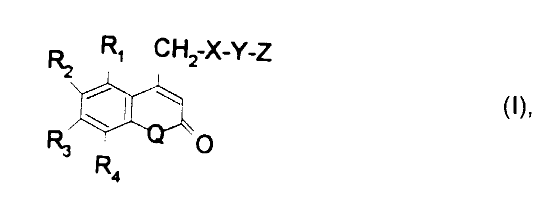 in welcher
- R₁: Wasserstoff oder C₁- bis C₆-Alkyl,
- R₂: Wasserstoff oder einen Rest OT₁ bedeuten oder
- R₁ und R₂: gemeinsam mit den Kohlenstoffatomen, an die sie gebunden sind, einen 3- bis 7-gliedrigen carbocyclischen Ring bilden,
- R₃: Wasserstoff oder einen Rest OT₂,
- R₄: Wasserstoff oder C₁- bis C₆-Alkyl,
- T₁ und T₂,: die gleich oder verschieden sind, jeweils Wasserstoff, C₁- bis C₆-Alkyl, C₁- bis C₆-Alkanoyl bedeuten oder T₁ und T₂ gemeinsam mit den Atomen, an die sie gebunden sind, einen 5- bis 7-gliedrigen heterocyclischen Ring bilden, der gewünschtenfalls mit Oxo oder Thioxo substituiert sein kann, oder
- R₃ und R₄: gemeinsam mit den Kohlenstoffatomen, an die sie gebunden sind, einen Pyridinring bilden,
- Q: ein Sauerstoffatom,
- X: eine Valenzbindung, ein Sauerstoff- oder Schwefelatom oder eine NH-Gruppe,
- Y: eine Valenzbindung, einen C₁- bis C₆-Alkylenrest, der gewünschtenfalls mit einer Hydroxygruppe oder einer Aminogruppe substituiert sein kann, einen Phenylenrest, der gewünschtenfalls ein- oder mehrfach mit Hydroxyl, Halogen, C₁- bis C₆-Alkyl oder Carboxyl substituiert sein kann,
- Z: Wasserstoff, Halogen, Carboxyl, Hydroxymethyl, C₁- bis C₆-Alkoxycarbonyl, Cyano oder eine Gruppe NR₅R₆ bedeuten, wobei
- R₅ und R₆,: die gleich oder verschieden sind, jeweils Wasserstoff oder C₁bis C₆-Alkyl darstellen oder
- R₅ und R₆: gemeinsam mit dem Stickstoffatom, an das sie gebunden sind, einen 3- bis 7-gliedrigen heterocyclischen Ring bilden, der gewünschtenfalls mit Oxo, Hydroxy oder C₁- bis C₆-Alkoxy substituiert sein kann oder ein weiteres Heteroatom wie Sauerstoff oder Schwefel enthält,
deren Tautomere sowie deren Salze mit nichttoxischen Säuren oder Basen.

Verbindungen der Formell sind zum Teil bekannt. So sind z.B. in Chem.Abstr. 60, 4114b Verbindungen der Formell mit R₁ = R₄ = H, R₂ = R₃ = OH, Q = O, X = Y = Valenz und Z=NR₅R₆ als Mittel zur Behandlung von Gefäßerkrankungen beschrieben. In Chem.Abstr. 84, 159773k wird angegeben, daß solche Substanzen keine Hemmung des Carrageninödems bewirkten.

Verbindungen mit R₁ = R ₄ = H, R₂ und R₃ = OH oder Alkyl = OH, Q = O, X = Valenz, Y = Alkylen und Z=Wasserstoff werden z.B. in Chem.Abstr. 68, 76748z als Inhibitoren der Phenylalaninhydrolase beschrieben, Verbindungen mit R₁ = R ₄ = H, R₂ und R₃ gemeinsam Methylendioxy oder R₂ = R₃ = OCH₃, Q = O, X = Y = Valenz und Z = Carboxyl oder Alkoxycarbonyl in Chem.Abstr. 100, 31674k und 112, 151027w als Fluoreszenzmarker.

Die Synthese von 4-Methyl-6,7-dihydroxycumarin ist in Chem.Ber. 34, 423 (1901) beschrieben. Aus Chem.Abstr. 60, 4113 (1964) bzw. Beilstein 18/3 V 233 ist 4-Chlormethyl-6,7-dihydroxycumarin bekannt.

In FR-A-2 276 819 sind 4-Methylcumarin-Derivate mit Acyloxy-Substituenten in 6,7-Stellung beschrieben. Diese Verbindungen zeigen im PLA₂-Test keine Hemmung. In GB-A-1 226 896 sind 4-Aminomethyl-7-hydroxycumarine als Choleretica beschrieben.

In EP-A-0 164 040 sowie J. Med. Chem. Bd. 34, No. 7, 1991, Seiten 2268-2274, ist die Verbindung 4-(2-aminoethyl)methyl-7-methoxycumarin als Zwischenprodukt beschrieben.

In Chem. Pharm. Bull Bd. 30, Nr. 11, 1982, Seiten 4120-4125, sind 4-Hydroxymethylcumarin-Derivate als Fluoreszenz-Marker beschrieben.

In Chem. Abstr. 92: 41 697a sind Verbindungen mit der Gruppe X-Y-Z = NH₂ als Fluoreszenz-Marker beschrieben.

In Chem. Abstr. 95: 150 350q, 98: 143 233n, 103: 141 793t und 104: 168 327b sind 6- oder 7-Hydroxy-Cumarine als antibakterielle Mittel bzw. Fluoreszenz-Marker beschrieben.

In Chem. Abstr. 100: 3167k ist die Verbindung 4-Hydroxymethyl- und 4-Brommethyl-6,7-dimethoxycumarin als Fluoreszenz-Marker beschrieben.

Eine antientzündliche Wirkung oder speziell ein PLA₂-Inhibitor ist nicht aufgeführt.

Die Verbindungen der Formell weisen wertvolle pharmakologische Eigenschaften auf, insbesondere können sie die Aktivität von Phospholipasen hemmen. Sie eignen sich daher zur Behandlung akuter und chronischer, allergischer, nichtallergischer und traumatischer und entzündlicher Erkrankungen, wie beispielsweise rheumatische Arthritis, Osteoarthritis, ulcerative Colitis, akute Pankreatitis, Kontaktdermatitis, entzündliche und allergische Atemwegserkrankungen, septischer Schock, allergischer Schock, Serumkrankheit, Autoimmunerkrankungen, graft-versus-host-Reaktionen, host-versus-graft-Erkrankungen, ischämische oder thrombotische Erkrankungen, beispielsweise Coronarinfarkt oder Cerebralinfarkt.

Alkylreste in den genannten Gruppen Alkyl, Alkoxy und Alkanoyl können geradkettig oder verzweigt sein. Bevorzugte Reste sind der Methyl-, Ethyl-, Propyl-, Isopropyl-, n-Butyl-, Isobutyl-, tert.-Butyl-, n-Pentyl- und 3-Pentylrest.

Alkylenreste können geradkettig oder verzweigt sein. Bevorzugte Reste sind Methylen, 1,2-Ethylen, 1,1-Ethylen, 1,3-Propylen, 1,2-Propylen und 2,2-Propylen.

Halogenatome sind insbesondere Fluor, Chlor und Brom.

Reste, die R₁ und R₂ zusammen einen carbocyclischen Ring bilden lassen, sind bevorzugt:
-CH₂-CH₂-CH₂-, -CH₂-CH₂-CH₂-CH₂- oder CH=CH-CH=CH-·

Reste, die R₂ und R₃ zusammen einen heterocyclischen Ring bilden lassen, sind bevorzugt:

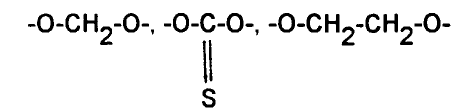

Reste, die R₅ und R₆ zusammen mit dem Stickstoffatom einen heterocyclischen Ring bilden lassen, sind bevorzugt:
-CH₂-CH₂-CH₂-CH₂, -CH₂-CH₂-CH₂-CH₂-CH₂-,
-CH₂-CH₂-O-CH₂-CH₂-, -CH₂-CH₂-S-CH₂-CH₂-

Außer den in den Beispielen genannten Verbindungen sind Gegenstand der Erfindung insbesondere alle Substanzen, die jede mögliche Kombination der in den Beispielen genannten Substituenten aufweisen.

Gegenstand der Erfindung sind auch neue Verbindungen der Formel I, in denen X nicht eine Valenzbindung darstellt, sowie deren Salze mit nichttoxischen Säuren oder Basen.

Das erfindungsgemäße Verfahren zur Herstellung der Verbindungen der Formell ist dadurch gekennzeichnet, daß man in an sich bekannte Weise eine Verbindung der allgemeinen Formel II 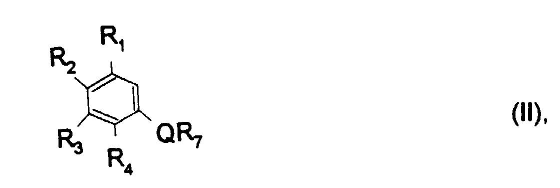 in welcher R₁ bis R₄ sowie Q die obengenannte Bedeutung haben und R₇ für Wasserstoff oder einen C₁- bis C₆-Alkanoylrest steht,
mit einer Verbindung der allgemeinen Formel III 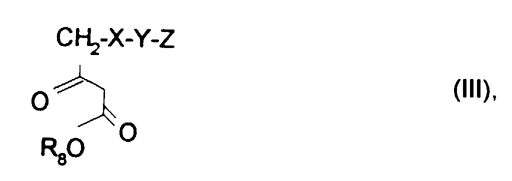 in welcher X, Y und Z die obengenannte Bedeutung haben und R₈ für einen C₁- bis C₆-Alkylrest steht, umsetzt und cyclisiert,
anschließend gewünschtenfalls einen oder mehrere Reste R₁ bis R₄, X, Y oder Z in jeweils einen anderen, durch den Anspruch definierten Rest umwandelt sowie gewünschtenfalls durch Neutralisation mit nichttoxischen Säuren oder Basen in ein Salz umwandelt.

Die Umsetzung von Verbindungen der Formel II mit Verbindungen der Formel III erfolgt zweckmäßig in saurem Medium wie beispielsweise verdünnter Schwefelsäure oder in einem nichtwäßrigen Lösungsmittel in Gegenwart einer Lewis-Säure, wie z.B. Zinkchlorid oder Aluminiumchlorid, unter Erwärmung.

Eine gewünschtenfalls anschließende Umwandlung eines oder mehrerer Reste bedeutet beispielsweise
- eine Abspaltung eines für T₁ und/oder T₂ stehenden Alkyl- oder Alkanoylrestes durch Etherspaltung bzw. Hydrolyse oder dessen Einführung durch Alkylierung bzw. Veresterung,
- der Ersatz eines für XYZ stehenden Halogenatoms durch nucleophilen Austausch unter Bildung einer O-YZ-, NH-YZ- oder S-YZ-Gruppe, einer CN-Gruppe oder einer Gruppe NR₅R₆,
- die Verseifung einer für Z stehenden Alkoxycarbonyl- oder Cyanogruppe zu einer Carboxylgruppe,
- die Veresterung einer für Z stehenden Carboxylgruppe zu einer Alkoxycarbonylgruppe,
- die Reduktion einer Alkoxycarbonyl- oder einer Carboxylgruppe zu einer Hydroxymethylgruppe.

Die Verbindungen der Formeln II und III sind literaturbekannt oder können auf triviale Weise aus bekannten Vorstufen synthetisiert werden.

Als pharmakologisch verträgliche Salze kommen insbesondere Alkali-, Erdalkaliund Ammoniumsalze sowie gegebenenfalls Salze mit nichttoxischen anorganischen oder organischen Säuren, wie z.B. Salzsäure, Schwefelsäure, Phosphorsäure, Bromwasserstoffsäure, Essigsäure, Milchsäure, Zitronensäure, Äpfelsäure, Benzoesäure, Salicylsäure, Malonsäure, Maleinsäure, Bemsteinsäure oder Diaminocapronsäure in Frage.

Die Salze erhält man in üblicher Weise z.B. durch Neutralisation der Verbindungen der Formell mit den entsprechenden Laugen oder Säuren.

Zur Herstellung von Arzneimitteln werden die Verbindungen der allgemeinen Formell in an sich bekannter Weise mit geeigneten pharmazeutischen Trägersubstanzen, Aroma-, Geschmacks- und Farbstoffen gemischt und beispielsweise als Tabletten oder Dragees ausgeformt oder unter Zugabe entsprechender Hilfsstoffe in Wasser oder Öl, wie z.B. Olivenöl, suspendiert oder gelöst.

Die Substanzen der allgemeinen Formell können in flüssiger oder fester Form oral und parenteral appliziert werden. Als Injektionsmedium kommt vorzugsweise Wasser zur Anwendung, welches die bei Injektionslösungen üblichen Stabilisierungsmittel, Lösungsvermittler und/oder Puffer enthält. Derartige Zusätze sind z.B. Tartrat- oder Borat-Puffer, Ethanol, Dimethylsulfoxid, Komplexbildner (wie Ethylendiamintetraessigsäure), hochmolekulare Polymere (wie flüssiges Polyethylenoxid) zur Viskositätsregulierung oder Polyethylen-Derivate von Sorbitanhydriden.

Feste Trägerstoffe sind z.B. Stärke, Lactose, Mannit, Methylcellulose, Talkum, hochdisperse Kieselsäure, höhermolekulare Polymere (wie Polyethylenglykole).

Für die orale Applikation geeignete Zubereitungen können gewünschtenfalls Geschmacks- und Süßstoffe enthalten. Für die äußerliche Anwendung können die erfindungsgemäßen Substanzenl auch in Form von Pudern und Salben verwendet werden. Sie werden dazu z.B. mit pulverförmigen, physiologisch verträglichen Verdünnungsmitteln bzw. üblichen Salbengrundlagen vermischt. Die verabreichte Dosis hängt vom Alter, der Gesundheit und dem Gewicht des Empfängers, dem Ausmaß der Krankheit, der Art gleichzeitiger gegebenenfalls durchgeführter weiterer Behandlungen, der Häufigkeit der Behandlungen und der Art der gewünschten Wirkung ab. Üblicherweise beträgt die tägliche Dosis der aktiven Verbindung 0.1 bis 50mg/kg Körpergewicht. Normalerweise sind 0.5 bis 40 und vorzugsweise 1.0 bis 20mg/kg/Tag in einer oder mehreren Anwendungen pro Tag wirksam, um die gewünschten Resultate zu erhalten.

Außer den in den Beispielen genannten Substanzen sind im Sinne der Erfindung die folgenden Verbindungen bevorzugt:
- 6,7-Dihydroxycumarin-4-essigsäure
   [Ausb. 35 %, Schmp. 210-212 °C (Wasser)]
- 6,7-Dihydroxy-4-(2-hydroxy-ethyl)cumarin
- 3-(6,7-Dihydroxycumarin-4-yl)propionsäure
- 3-(2-Oxo-2H-pyrano [3,2-g] chinolin-4-yl-methylmercapto)propionsäure
- 3-(6-Hydroxy-3-oxo-3,7,8,9-tetrahydro-cyclopenta [f]
   [1]benzopyran-1-yl-methylmercapto)propionsäure
- 3-(6-Hydroxy-3-oxo-7,8,9,10-tetrahydro-3H-naphtho
   [2,1-b]pyran-1-yl-methylmercapto)propionsäure
- 3-(6-Hydroxy-3-oxo-3H-naphtho
   [2,1 - b]pyran-1-yl-methylmercapto)propionsäure
- 3-(5-Allyl-6-hydroxy-3-oxo-3H-naphtho [2,1-b] pyran-1-yl- methylmercapto)propionsäure
- 3-(6-Hydroxy-3-oxo-5-propyl-3H-naphtho [2,1 - b]pyran-1-yl- methylmercapto)propionsäure

### Beispiel 1

### 4-Methyl-6,7-dihydroxycumarin (Referenz-Verbindung)

Zu einer Mischung aus 5.8ml Acetessigsäureethylester und 11.4g 1,2,4-Triacetoxybenzol tropft man 45ml 75%ige Schwefelsäure, erwärmt 30Min. auf 80°C, gießt auf Eis, filtriert, wäscht den Niederschlag neutral und trocknet im Vakuum. Man erhält 8,2g Titelverbindung (95% d.Th.) vom Schmelzp. 270 bis 272°C.

### Beispiel 2

In analoger Weise wie in Beispiel 1 beschrieben erhält man:

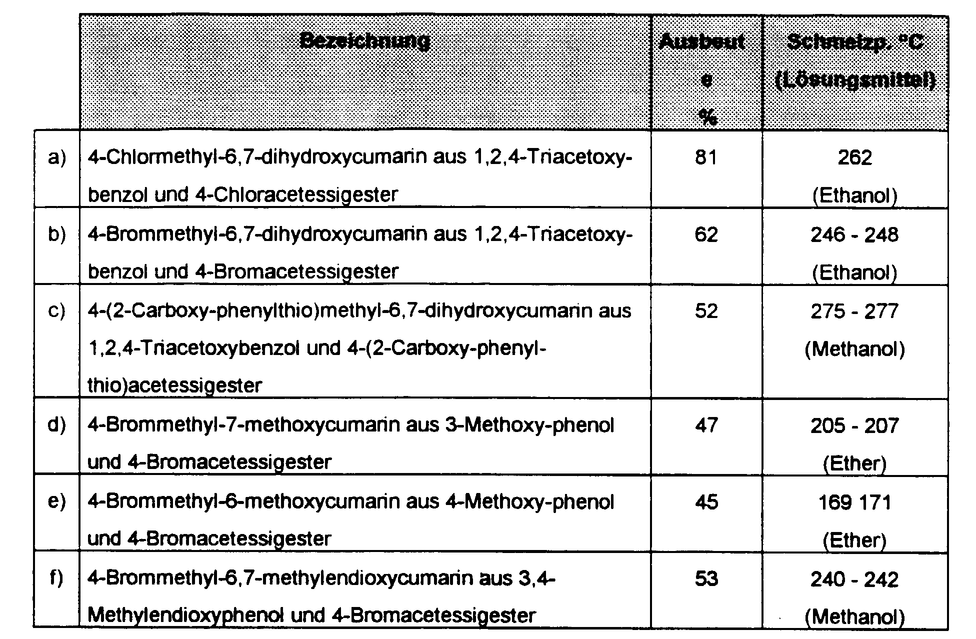

### Beispiel 3

### 4-N,N-Dimethylaminomethyl-6,7-dihydroxycumarin

Zu einer Lösung von 4,5 g Dimethylamin in 300ml Methanol gibt man 5,7g Verbindung des Beispiels2a, rührt über Nacht bei Raumtemperatur, filtriert, engt ein und chromatographiert an Kieselgel (Elutionsmittel Ethylacetat: Methanol 9:1). Man isoliert 3,3g Titelverbindung (56% d.Th.) vom Schmelzp. 212 bis 215°C.

### Beispiel 4

In analoger Weise wie in Beispiel 3 beschrieben erhält man:

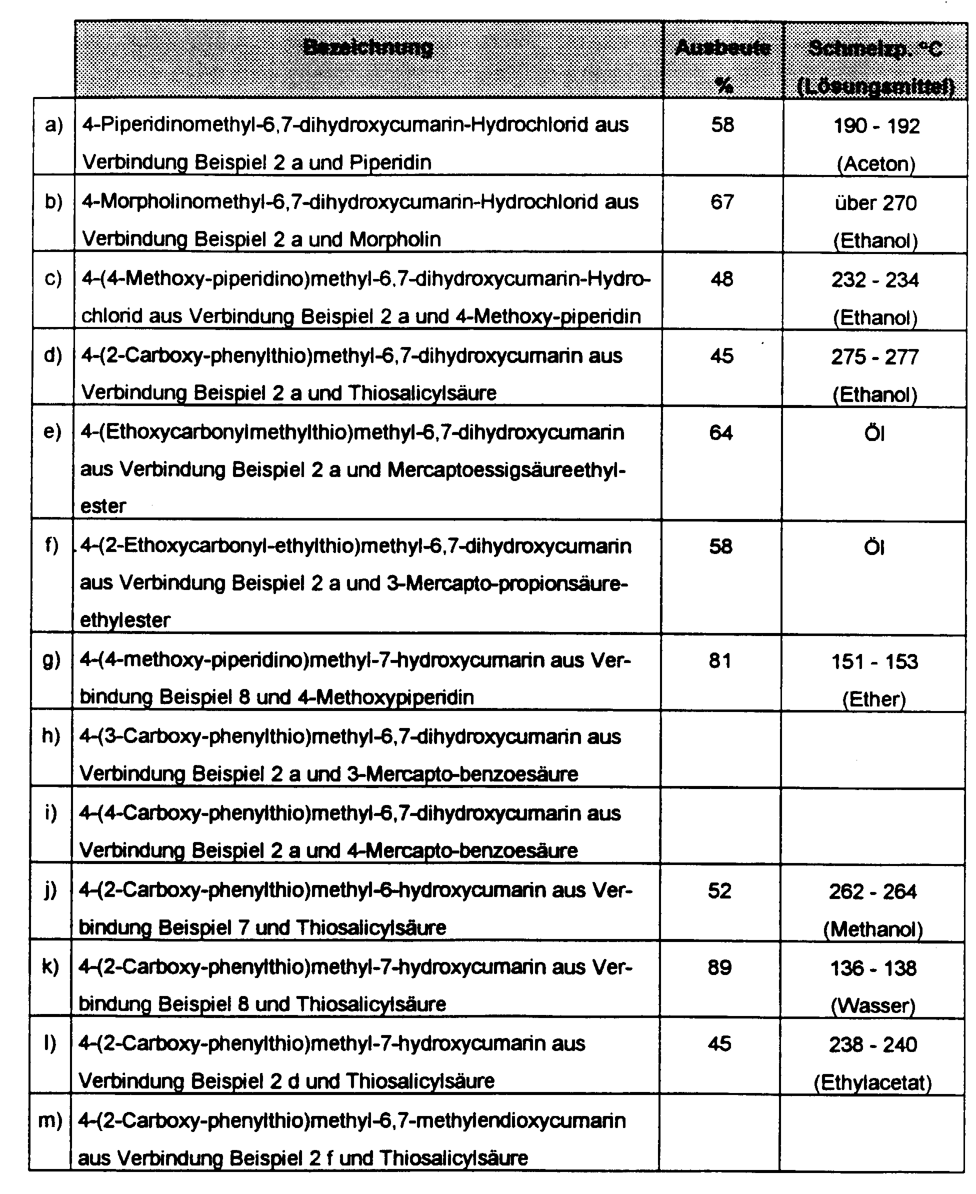

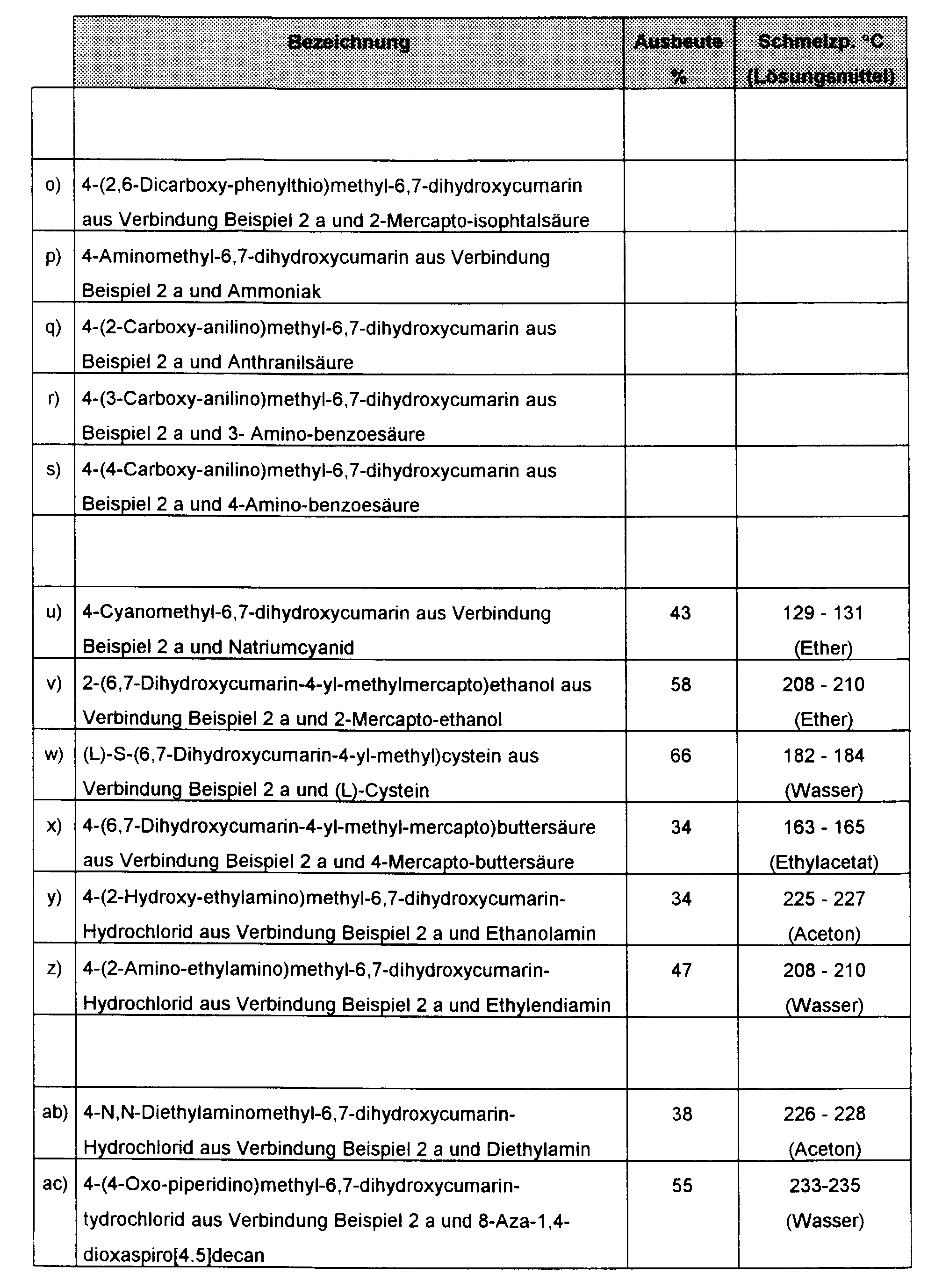

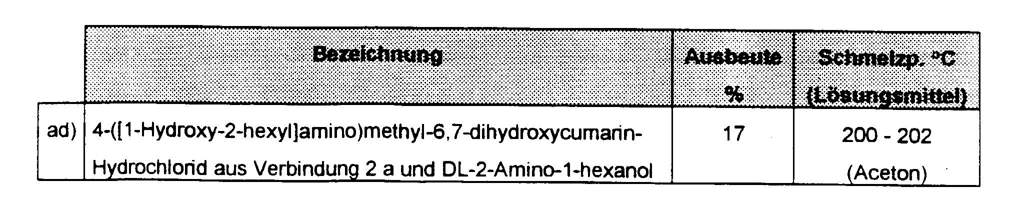

### Beispiel 5

### 6,7-Dihydroxycumarin-4-yl-methylmercaptoessigsäure

Eine Mischung aus 3.1g Verbindung des Beispiels 4e und 300ml 50%ige Essigsäure wird 24h zum Rückfluß erhitzt. Man engt ein, nimmt in verd. Natriumhydrogencarbonatlösung auf, wäscht mit Ethylacetat, säuert die wäßrige Phase an und filtriert das Produkt ab. Es verbleiben 2.1g Titelverbindung (74% d. Th.) vom Schmelzp. 254 - 256°C.

### Beispiel 6

In analoger Weise wie in Beispiel 5 beschrieben erhält man:

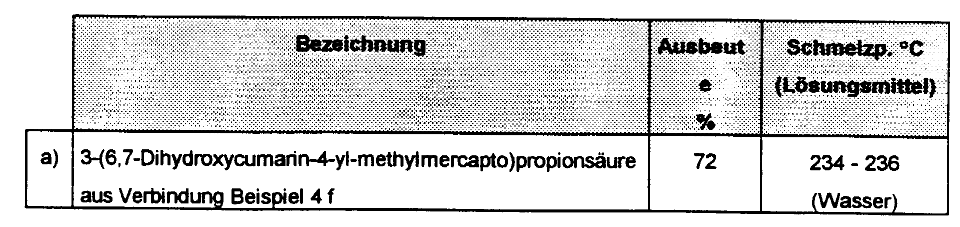

### Beispiel 7

### 4-Brommethyl-6-hydroxycumarin

Eine Mischung aus 2.7g Verbindung des Beispiels 2e, 100ml Dichlormethan und 5.6ml Bortribromid wird 4h bei Raumtemperatur gerührt, auf Eis gegossen und das Produkt abfiltriert. Man isoliert 1.8g Titelverbindung (71% d. Th.) vom Schmelzp. 198 bis 200°C.

### Beispiel 8

In analoger Weise, wie in Beispiel 7 beschrieben, erhält man 4-Brommethyl-7-hydroxycumarin aus Verbindung Beispiel 2d in 70% Ausbeute vom Schmelzp. 182 bis 184 °C.

### Beispiel 9

### 6,7-Diacetoxy-4-(4-methoxy-piperidino)methylcumarin-Hydrochlorid

Eine Mischung aus 1.5g Verbindung des Beispiels 4c und 15ml Essigsäureanhydrid wird 2h zum Rückfluß erhitzt. Man engt ein, verreibt mit Ether und filtriert das Produkt ab. Es verbleiben 1.4g Titelverbindung (75% d. Th.) vom Schmelzp. 206 bis 208°C.

### Beispiel 10

### 6,7-Dimethoxy-4-(4-methoxy-piperidino)methylcumarin

Zu einer Lösung von 1.5g Verbindung des Beispiels 4c in 6 ml 2N Natronlauge gibt man 1.1ml Dimethylsulfat und rührt über Nacht bei Raumtemperatur. Man extrahiert mit Dichlormethan, trocknet und engt den Extrakt ein. Es verbleiben 0,44g Titelverbindung (30% d. Th.) vom Schmelzp. 148 bis 150°C.

### Beispiel 11

### 8-(4-Methoxy-piperidino)methyl-2-thioxo-1,3-dioxolo[4,5-g]cumarin

Zu einer Mischung aus 1.5g Verbindung des Beispiels 4c und 40 ml Chloroform gibt man 5ml Pyridin und tropft bei 20°C 1.12ml Thiophosgen, gelöst in 10ml Chloroform, zu. Man rührt über Nacht bei Raumtemperatur, wäscht die Lösung mit Wasser, trocknet die organische Phase und reinigt an Kieselgel. Mit Ethylacetat eluiert man 1,5g Titelverbindung (88% d. Th.), die nach Verreiben mit Ether bei 108 bis 110°C schmelzen.

### Beispiel 12

In analoger Weise, wie in Beispiel 9 beschrieben, erhält man aus Verbindung Beispiel 2c in 68%iger Ausbeute 4-(2-Carboxy-phenylthio)methly-6,7-diacetoxycumarin. Schmp. 216-218 °C (Ethylacetat).

### Beispiel 13

In analoger Weise, wie in Beispiel 10 beschrieben, erhält man aus Verbindung Beispiel 2c in 45%iger Ausbeute 4-(2-Carboxy-phenylthio)methyl-6,7-dimethoxycumarin.

### Beispiel 14

In analoger Weise, wie in Beispiel 10 beschrieben, erhält man aus Verbindung Beispiel 2c und 1,2-Dibromethan 4-(2-Carboxy-phenylthio)methyl-6,7-ethylendioxycumarin.

### Pharmakologische Tests

### Wirkungen der Cumarine

Die vorliegende Anmeldung betrifft Arzneimittel zur Behandlung akuter und chronischer Erkrankungen mit entzündlicher, immunologischer, allergischer, nicht-allergischer oder traumatischer Genese.

Da die Phospholipase A₂ (PLA₂) ein Schlüsselenzym bei der Entstehung dieser Erkrankungen ist, werden durch ihre Hemmung diese Erkrankungen wirkungsvoll bekämpft.

Die Cumarine sind nun Inhibitoren der PLA₂, die in ihrem Wirkprofil den eingeführten antiphlogistischen Prinzipien wie z. B. Cyclooxygenaseinhibitoren überlegen sind. Zum Vergleich der Wirkprofile wurde mit Indometacin ein typischer Cyclooxygenaseinhibitor ausgewählt. Die Abnahme der enzymatischen Aktivität, gemessen an der Abspaltung von Fettsäuren aus der Position sn-2 von Lecithin, wurde als Indikator der PLA₂-Hemmung gemessen.

### Hemmung der PLA₂-Aktivität

Als typischer Repräsentant einer PLA₂ wurde die humane rekombinante Typ-II-PLA₂ (= synoviale PLA₂) zur Testung eingesetzt.

Die Tabelle 1 enthält die prozentuale in vitro-Hemmung dieses Enzyms durch die beispielhaften Verbindungen Bsp. 4 c), Bsp. 5 und Bsp. 4 w). Das Enzym wurde dosisabhängig bis zu 100 % gehemmt.

Demgegenüber sieht man in Tabelle 1, daß Indometacin das Enzym zwar auch hemmte, jedoch nur mit maximal 52 % bei der höchsten Konzentration. Hierin dokumentiert sich die Überlegenheit der neuen sPLA₂-Inhibitoren gegenüber einem Cyclooxygenasehemmer.

**Tabelle 1:**

| Hemmung der sPLA2-Enzymaktivität durch Cumarinderivate und Indometacin | | | | |
|---|---|---|---|---|
| **Konzentrationen** | **Substanzen** | | | |
| | **Bsp. 4 c)** | **Bsp. 5** | **Bsp. 4 w)** | **Indometacin** |
| 100 µg/ml | 86 | n.b. | n.b. | 52 |
| 10 µg/ml | 89 | 97 | 95 | 10 |
| 1 µg/ml | 34 | 49 | 37 | 0 |
| 0,1 µg/ml | 6 | 9 | 4 | n.b. |
| Mittelwerte aus 4 Versuchen (Doppelbestimmungen), n.b. = nicht bestimmt | | | | |

### Schlußfolgerung

Die geringere Hemmung der humanen rekombinanten Typ-II-PLA₂ durch Indometacin veranschaulicht, daß dieser Cyclooxygenasehemmer nur eine schwache PLA₂-inhibierende Wirkung besitzt. Demgegenüber hemmten die Cumarin-Vertreter Bsp. 4 c), Bsp. 5 und Bsp 4 w) die PLA₂ dosisabhängig und bis zu 100 % und sind hierdurch eingeführten entzündungshemmenden Prinzipien überlegen.

## Patentansprüche

1. Verwendung von Verbindungen der Formel I 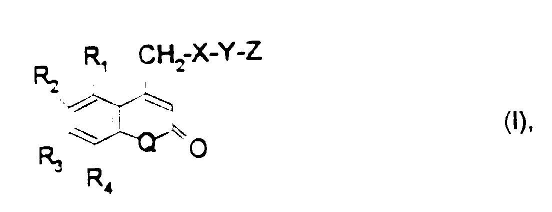 in weicher
R₁ Wasserstoff oder C₁- bis C₆-Alkyl,
R₂ Wasserstoff oder einen Rest OT₁ bedeuten oder
R₁ und R₂ gemeinsam mit den Kohlenstoffatomen, an die sie gebunden sind, einen 3- bis 7-gliedrigen carbocyclischen Ring bilden,
R₃ Wasserstoff oder einen Rest OT₂,
R₄ Wasserstoff oder C₁- bis C₆-Alkyl,
T₁ und T₂, die gleich oder verschieden sind, jeweils Wasserstoff, C₁- bis C₆-Alkyl, C₁- bis C₆-Alkanoyl bedeuten oder T₁ und T₂ gemeinsam mit den Atomen. an die sie gebunden sind, einen 5- bis 7-gliedrigen heterocyclischen Ring bilden, der gewünschtenfalls mit Oxo oder Thioxo substituiert sein kann, oder
R₃ und R₄ gemeinsam mit den Kohlenstoffatomen, an die sie gebunden sind, einen Pyridinring bilden,
Q ein Sauerstoffatom
X eine Valenzbindung, ein Schwefelatom oder eine NH-Gruppe,
Y eine Valenzbindung, einen C₁- bis C₆-Alkylenrest, der gewünschtenmit einer Hydroxygruppe oder einer Aminogruppe substituiert sein kann, einen Phenylenrest, der gewünschtenfalls ein- oder mehrfach mit Hydroxyl, Halogen, C₁- bis C₆-Alkyl oder Carboxyl substituiert sein kann.
Z Halogen. Carboxyl. Hydroxymethyl, C₁- bis C₆-Alkoxycarbonyl, Cyano oder eine Gruppe NR₅R₆ beaeuten, wobei
R₅ und R₆, die gleich oder verschieden sind, jeweils Wasserstoff oder C₁ bis C₆-Alkyl darstellen oder
R₅ und R₆ gemeinsam mit dem Stickstoffatom, an das sie gebunden sind, einen 3- bis 7-gliedrigen heterocyclischen Ring bilden, der gewünschtenfalls mit Oxo, Hydroxy oder C₁- bis C₆-Alkoxy substituiert sein kann oder ein weiteres Heteroatom wie Sauerstoff oder Schwefel enthält.
deren Tautomere sowie deren Salze mit nichttoxischen Säuren oder Basen zur Herstellung von Arzneimitteln mit PLA₂-Inhibitor-Wirkung.

2. Verbindungen der Formel I 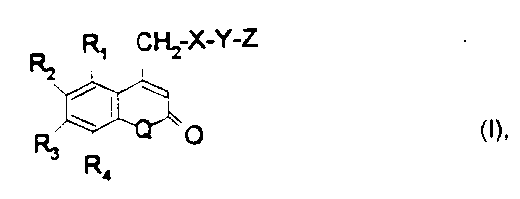 in welcher
R₁ Wasserstoff oder C₁- bis C₆-Alkyl,
R₂ einen Rest OT₁,
R₃ einen Rest OT₂,
R₄ Wasserstoff oder C₁- bis C₆-Alkyl,
T₁ und T₂, die gleich oder verschieden sind, jeweils Wasserstoff, C₁- bis C₆-Alkyl, C₁- bis C₆-Alkanoyl bedeuten oder T₁ und T₂ gemeinsam mit den Atomen, an die sie gebunden sind, einen 5- bis 7-gliedrigen heterocyclischen Ring bilden, der gewünschtenfalls mit Oxo oder Thioxo substituiert sein kann, oder
Q ein Sauerstoffatom,
X ein Schwefelatom oder eine NH-Gruppe,
Y eine Valenzbindung, einen C₁- bis C₆-Alkylenrest, der gewünschtenfalls mit einer Hydroxygruppe oder einer Aminogruppe substituiert sein kann, einen Phenylenrest, der gewünschtenfalls ein- oder mehrfach mit Hydroxyl, Halogen, C₁- bis C₆-Alkyl oder Carboxyl substituiert sein kann,
Z Wasserstoff, Halogen, Carboxyl, Hydroxymethyl, C₁- bis C₆-Alkoxycarbonyl, Cyano oder eine Gruppe NR₅R₆ bedeuten, wobei
R₅ und R₆ gemeinsam mit dem Stickstoffatom, an das sie gebunden sind, einen 3- bis 7-gliedrigen heterocyclischen Ring bilden, der mit Oxo, Hydroxy oder C₁- bis C₆-Alkoxy substituiert ist,
deren Tautomere sowie deren Salze mit nichttoxischen Säuren oder Basen.

3. 4-(4-Methoxy-piperidino)methyl-6,7-dihydroxycumarin, dessen Tautomeres sowie dessen Salze mit nichttoxischen Säuren oder Basen.

4. Verfahren zur Herstellung von Verbindungen der Formel I gemäß Anspruch 2 und der Verbindung gemäß Anspruch 3,
deren Tautomere sowie deren Salze mit nichttoxischen Säuren oder Basen, dadurch gekennzeichnet, daß man in an sich bekannter Weise eine Verbindung der Formel II 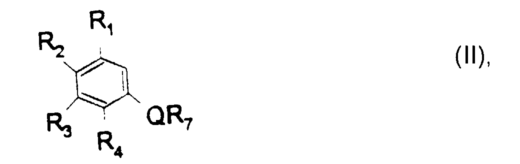 in welcher R₁ bis R₄ sowie Q die in Anspruch 2 oder 3 genannte Bedeutung haben und R₇ für Wasserstoff oder einen C₁- bis C₆-Alkanoylrest steht,
mit einer Verbindung der allgemeinen Formel III 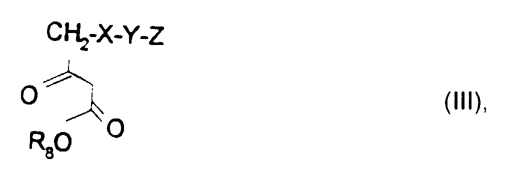 in welcher X, Y und Z die in Anspruch 2 oder 3 genannte Bedeutung haben und R₈ für einen C₁- bis C₆-Alkylrest steht, umsetzt und cyclisiert,
anschließend gewünschtenfalls einen oder mehrere Reste R₁ bis R₄, X, Y oder Z in jeweils einen anderen, durch den Anspruch definierten Rest umwandelt sowie gewünschtenfalls durch Neutralisation mit nichttoxischen Säuren oder Basen in ein Salz umwandelt.

5. Arzneimittel enthaltend mindestens eine Verbindung der Formel I gemäß Anspruch 2 oder 3 neben anderen üblichen Träger- und Hilfsstoffen.

## Claims

1. Use of compounds of formula I 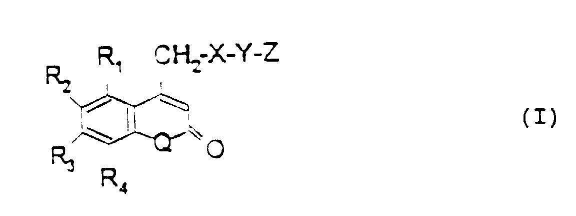 in which
R₁ denotes hydrogen or C₁ to C₆ alkyl,
R₂ denotes hydrogen or a residue OT₁ or
R₁ and R₂ together with the carbon atoms to which they are bound,form a 3-membered to 7-membered carbocyclic ring
R₃ denotes hydrogen or a residue OT₂
R₄ denotes hydrogen or C₁ to C₆ alkyl
T1 and T2 which can be the same or different in each case denote hydrogen C1 to C6 alkyl, C1 to C6 alkanoyl or T1 and T2 together with the atoms to which they are bound, form a 5-membered to 7-membered heterocyclic ring which can be substituted if desired with oxo or thioxo, or
R₃ and R₄ together with the carbon atoms to which they are bound, form a pyridine ring
Q denotes an oxygen atom
X denotes a valency bond, a sulphur atom or an NH group,
Y denotes a valency bond, a C₁ to C₆ alkylene residue which can be substituted if desired with a hydroxy group or an amino group, a phenylene residue which can be substituted if desired once or several times with hydroxyl, halogen, C₁ to C₆ alkyl or carboxyl
Z denotes halogen, carboxyl, hydroxymethyl, C₁ to C₆ alkoxycarbonyl, cyano or a group NR₅R₆ in which
R₅ and R₆ which can be the same or different each represent hydrogen or C₁ to C₆ alkyl or
R₅ and R₆ together with the nitrogen atom to which they are bound, form a 3-membered to 7-membered heterocyclic ring which can be substituted if desired with oxo, hydroxy or C₁ to C₆ alkoxy or contains a further heteroatom such as oxygen or sulphur
their tautomers as well as salts thereof with non-toxic acids or bases for the production of pharmaceutical preparations with a PLA₂-inhibiting effect.

2. Compounds of formula I 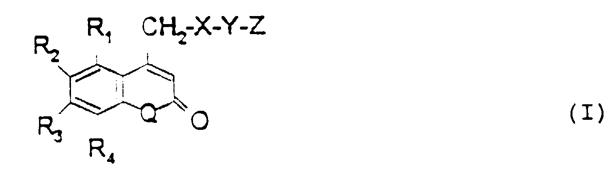 in which
R₁ denotes hydrogen or C₁ to C₆ alkyl
R₂ denotes a residue OT₁
R₃ denotes a residue OT₂,
R₄ denotes hydrogen or C₁ to C₆ alkyl,
T1 and T2 which can be the same or different in each case denote hydrogen C1 to C6 alkyl, C1 to C6 alkanoyl or T1 and T2 together with the atoms to which they are bound, form a 5-membered to 7-membered heterocyclic ring which can be substituted if desired with oxo or thioxo, or
Q denotes an oxygen atom
X denotes a sulphur atom or an NH group
Y denotes a valency bond, a C₁ to C₆ alkylene residue which can be substituted if desired with a hydroxy group or an amino group, a phenylene residue which can be substituted if desired once or several times with hydroxyl, halogen, C₁ to C₆ alkyl or carboxyl
Z denotes hydrogen, halogen, carboxyl, hydroxymethyl, C₁ to C₆ alkoxycarbonyl, cyano or a group NR₅R₆ in which
R₅ and R₆ together with the nitrogen atom to which they are bound, form a 3-membered to 7-membered heterocyclic ring which is substituted with oxo, hydroxy or C₁ to C₆ alkoxy
their tautomers as well as salts thereof with non-toxic acids or bases.

3. 4-(4-Methoxy-piperidino)methyl-6,7-dihydroxy-coumarin, tautomers thereof as well as salts thereof with non-toxic acids or bases.

4. Process for the production of compounds of formula I as claimed in claim 2 and of a compound as claimed in claim 3 their tautomers as well as salts thereof with non-toxic acids or bases, wherein a compound of formula II 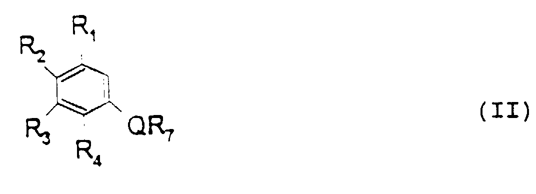 in which R₁ to R₄ and Q have the meanings stated above and R₇ represents hydrogen or a C₁ to C₆ alkanoyl residue
is reacted in a well-known manner with a compound of the general formula III 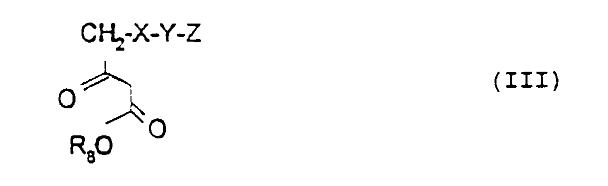 in which X, Y and Z have the meaning stated in claim 2 or 3 and R₈ represents a C₁ to C₆ alkyl residue and it is cyclized,
subsequently one or several residues R₁ to R₄, X, Y or Z are optionally converted into another residue defined by the claim and optionally into a salt by neutralizing with non-toxic acids or bases.

5. Pharmaceutical preparation containing at least one compound of formula I as claimed in claim 2 or 3 in addition to other conventional carrier and auxiliary substances.

## Revendications

1. Utilisation de composés de formule I 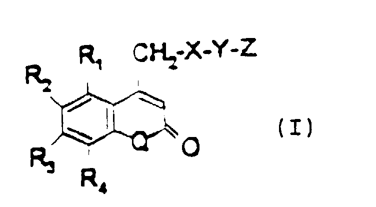 dans laquelle
R₁ représente un atome d'hydrogène ou un groupement alkyle en C₁ à C₆,
R₂ représente un atome d'hydrogène ou un résidu OT₁, ou
R₁ et R₂ forment conjointement avec les atomes de carbone auxquels ils sont liés un cycle carboné de 3 à 7 éléments,
R₃ représente un atome d'hydrogène ou un résidu OT₂,
R₄ représente un atome d'hydrogène ou un groupement alkyle en C₁ à C₆,
T₁ et T₂, qui sont identiques ou différents, représentent respectivement un atome d'hydrogène, un groupement alkyle en C₁ à C₆, un groupement alcanoyle en C₁ à C₆, ou bien T₁ et T₂ forment conjointement avec les atomes auxquels ils sont liés un hétérocycle qui peut être substitué à souhait par un groupement oxo ou thioxo, ou bien
R₃ et R₄ foprment conjointement avec les atomes de carbone auxquels ils sont liés, un cycle pyridine,
Q représente un atome d'oxygène,
X représente une liaison de valence, un atome de soufre ou un groupement NH,
Y représente une liaison de valence, un résidu alcényle en C₁ à C₆, qui peut être substitué à souhait par un groupement hydroxyle ou un groupement amine, un résidu phénylène, qui peut être mono- ou poly-substitué par un radical hydroxyle, halogéno, alkyle en C₁ à C₆ ou carboxyle,
Z représente un atome d'halogène, un groupement carboxyle, hydroxyméthyle, alcoxycarbonyle en C₁ à C₆, cyano ou un groupement NR₅R₆ dans lequel
R₅ et R₆ qui sont identiques ou différents représentent respectivement un atome d'hydrogène ou un groupement alkyle en C₁ à C₆ ou bien
R₅ et R₆ forment conjointement avec les atomes d'azote auxquels ils sont liés, un hétérocycle de 3 à 7 éléments qui peut substitué à souhait par un groupement oxo, hydroxy ou alcoxy en C₁ à C₆ ou qui contient un hétéro-atome supplémentaire comme l'atome d'oxygène ou de soufre,
leurs tautomères ainsi que leurs sels avec des acides ou des bases non toxiques pour la fabrication de médicaments ayant un effet d'inhibition de PLA.

2. Composés de formule I 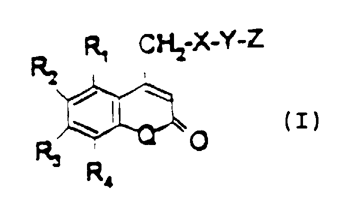 dans laquelle
R₁ représente un atome d'hydrogène ou un groupement alkyle en C₁ à C₆,
R₂ représente un résidu OT₁,
R₃ représente un résidu OT₂,
R₄ représente un atome d'hydrogène ou un groupement alkyle en C₁ à C₆
T₁ et T₂, qui sont identiques ou différents, représentent respectivement un atome d'hydrogène, un groupement alkyle en C₁ à C₆, un groupement alcanoyle C₁ à C₆, ou T₁ et T₂ forment conjointement avec les atomes auxquels ils sont liés, un hétérocycle de 5 à 7 éléments qui peut être substitué à souhait par un groupement oxo ou thioxo, ou bien
Q représente un atome d'oxygène,
X représente un atome de soufre ou un groupement NH,
Y représente une liaison de valence, un résidu alcényle en C₁ à C₆, qui peut être substitué à souhait par un groupement hydroxyle ou un groupement amine, un résidu phénylène, qui peut être mono- ou poly-substitué par un radical hydroxyle, halogéno, alkyle en C₁ à C₆ ou carboxyle,
Z représente un atome d'hydrogène, d'halogène, un groupement carboxyle, hydroxyméthyle, alcoxycarbonyle en C₁ à C₆, cyano ou un groupement NR₅R₆ dans lequel
R₅ et R₆ conjointement avec l'atome d'azote auquel ils sont liés, forment un hétérocycle de 3 à 7 éléments qui peut substitué à souhait par un groupement oxo, hydroxy ou alcoxy en C₁ à C₆,
leurs tautomères ainsi que leur sels avec des acides ou des bases non toxiques.

3. La 4-(4-méthoxy-pipéridino)méthyl-6,7-dihydroxy-coumarine, ses tautomères ainsi que ses sels avec des acides ou des bases non toxiques.

4. Procédé pour fabriquer les composés de formule selon la revendication 2 et le composé selon la revendication 3, leurs tautomères ainsi que leurs sels avec des acides ou des bases non toxiques, caractérisé en ce que l'on fait réagir d'une manière connue en soi en vue d'une cyclisation, un composé de formule II 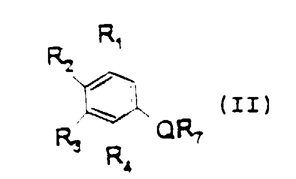 dans laquelle R₁ à R₄ ainsi que Q qui possède ladite signification selon la revendication 2 ou 3 et R₇ représente un atome d'hydrogène ou un résidu alcanoyle en C₁ à C₆, avec un composé de formule générale III 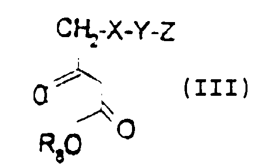 dans laquelle X, Y et Z ont ladite signification selon la revendication 2 ou 3 et R₈ représente un résidu alkyle en C₁ à C₆, ensuite à souhait un ou plusieurs résidus R₁ à R₄, X, Y ou Z, qui se transforment respectivement en un résidu défini par la revendication ainsi qu'à souhait en un sel par neutralisation avec des acides ou des bases non toxiques.

5. Médicament comprenant au moins un composé de formule I selon la revendication 2 ou 3 en plus d'autres substances véhicules et auxiliaires.
